# FASCICULE DE BREVET EUROPEEN

(11) **EP 0 841 549 B1**
(45) Date de publication et mention de la délivrance du brevet: **11.09.2002**
(21) Numéro de dépôt: 97118992.3
(22) Date de dépôt: 31.10.1997
(51) Int. Cl.: G01L 3/10, B60B 27/02, A61B 5/22

(54) **Moyeu de roue de cycle et roue équipée d'un tel moyeu**
Fahrradnabe und Rad mit solcher Nabe
Hub for cycle wheel and wheel with such a hub

(30) Priorité: 07.11.1996 FR 9613847
(43) Date de publication de la demande: 13.05.1998
(60) Demande divisionnaire: 98116781.0
(73) Titulaire: MAVIC S.A., 01990 Saint-Trivier sur Moignans (FR)
(72) Inventeur: Kubacsi, Michel, 01600 Toussieux (FR); Mercat, Jean-Pierre, 01990 Saint Trivier Sur Moignans (FR)
(74) Mandataire: Lejeune, Benoit

(56) Documents cités:
- EP-A- 0 393 427
- EP-A- 0 703 376
- DE-A- 3 150 149
- US-A- 4 811 612
- US-A- 4 966 380
- US-A- 5 016 478
- US-A- 5 018 392
- US-A- 5 031 455

## Description

L'invention concerne un moyeu de roue de cycle équipé d'un mécanisme d'entraînement, notamment un moyeu arrière. L'invention concerne également une roue de cycle comprenant un tel moyeu.

Parmi les moyeux arrière, l'invention concerne plus particulièrement ceux qui permettent la mesure du couple moteur transmis par la chaîne, c'est-à-dire par le cycliste dans le cas d'une bicyclette.

Dans ce cas, un tel dispositif renseigne son utilisateur sur la puissance instantanée qu'il développe. Ceci peut être utile par exemple dans le cadre d'un programme d'entraînement de l'athlète pour doser les efforts aux moments voulus. Ceci peut être utilisé aussi en compétition, pour aider le coureur à se placer dans des conditions optimales de dépenses physiques. D'autres utilisations existent, par exemple pour des programmes de rééducation.

Il est courant de mesurer la puissance dépensée par un sportif ou par une personne en rééducation sur des ergomètres et des bicyclettes d'intérieur. Dans ce cas, on mesure simplement la puissance dissipée dans un organe de freinage ou autre.

Le problème est différent ici. En effet, une bicyclette est un engin mobile mu par la puissance développée par le cycliste. La mesure de puissance doit être faite sans dissipation ou perte d'énergie. Autrement les performances propres de la bicyclette elle-même seraient affectées.

Un moyeu permettant la mesure de couple est par exemple connu de la demande de brevet allemand publiée sous le numéro DE 31 50 149. Ce dispositif comprend un corps de moyeu supportant les rayons et la jante, et un tube central de transmission portant les pignons d'entraînement de la chaîne. Une liaison élastique relie ce tube central et le corps de moyeu, et un dispositif de lecture mesure le décalage angulaire entre les deux éléments. Ce dispositif pose des problèmes au niveau de la réalisation pratique. En effet, la liaison élastique apparaît comme l'élément principal de la mesure de couple. Il est difficile d'étalonner de façon fiable un tel élément, c'est-à-dire de trouver un élément dont l'élasticité est invariable dans le temps, et insensible aux conditions d'utilisation. En outre, la construction est relativement complexe car le corps de moyeu est monté en rotation libre sur le tube central de transmission, lui-même monté en rotation libre sur un arbre.

Un autre moyeu permettant la mesure du couple d'entraînement est connu par la demande de brevet européen publiée sous le numéro EP 0 393 427. Comme le moyeu précédent, ce moyeu comprend un corps de moyeu monté sur un tube central de transmission. Le tube central porte les pignons d'entraînement à l'une de ses extrémités, et son autre extrémité est reliée solidairement au corps de moyeu. Sous l'action du couple d'entraînement, le tube central de transmission se déforme en torsion. Comme l'angle de rotation relative entre les deux extrémités du tube est relativement faible, on amplifie ce décalage angulaire au moyen d'un petit levier monté basculant.

Une fois encore, la construction est relativement complexe, étant donné que le moyeu est monté en rotation libre par rapport au tube de transmission au moins du côté des pignons.

Du côté des pignons, l'isolation en rotation du tube de transmission et du moyeu est difficile à réaliser. A ce niveau, il existe également des problèmes d'étanchéité. A l'autre extrémité du tube, de transmission, la liaison par encastrement du tube de transmission au moyeu est particulièrement délicate à réaliser. Aux deux extrémités, le moindre jeu de fonctionnement ou la moindre élasticité fausse la mesure angulaire et induit des erreurs significatives. Il apparaît également que le levier basculant réalise une amplification qui n'est pas linéaire.

Un but de l'invention est de proposer un moyeu permettant une mesure du couple d'entraînement dont la construction est plus simple. Un autre but de l'invention est de proposer un moyeu permettant une mesure plus fiable, c'est-à-dire diminuant les sources d'erreur, et présentant une linéarité améliorée.

Le moyeu de roue, notamment de roue de cycle, selon l'invention comprend un arbre transversal prévu pour être assemblé de façon solidaire sur un cadre, et définissant un axe transversal de révolution, un corps de moyeu monté en rotation libre autour de l'arbre transversal, le corps de moyeu ayant une partie médiane, deux joues latérales, les joues latérales ayant des moyens d'accrochage prévus pour des moyens de liaison avec une jante, le corps de moyeu ayant au-delà de l'une des joues un embout latéral sur lequel est monté un mécanisme de roue libre prévu pour recevoir au moins un pignon d'entraînement.

Le moyeu est caractérisé par le fait que la partie médiane du corps de moyeu est conformée en corps d'épreuve déformable en torsion autour de l'axe transversal de révolution, et un couple de deux capteurs est disposé dans le moyeu, les capteurs étant prévus pour mesurer au moins de façon séquentielle le décalage angulaire entre les deux extrémités du moyeu par rapport à l'axe transversal de révolution.

La roue de cycle selon l'invention comprend un moyeu central avec un arbre transversal définissant un axe transversal, un corps de moyeu monté en rotation libre autour de l'arbre, avec une partie médiane et deux joues latérales et un embout prévu pour recevoir au moins un pignon de transmission et un mécanisme de roue libre unidirectionnelle, une jante, et deux nappes de rayons reliant la jante à chacune des joues du moyeu.

Elle est caractérisée par le fait que la partie médiane du corps de moyeu est conformée comme un corps d'épreuve déformable en torsion, que les nappes de rayons sont prévues pour que la nappe de rayons située du côté opposé à l'embout soit destinée à transmettre une partie déterminée du couple d'entraînement transmis à l'embout par le pignon et le mécanisme de roue libre, et que le moyeu présente des capteurs prévus pour mesurer la position angulaire relative de chacune de ses joues.

Ainsi, dans le cas présent, c'est le corps de moyeu lui-même dont on mesure la déformation en torsion. De plus, on favorise cette torsion en maîtrisant entre les deux nappes de rayons la répartition du couple d'entraînement qui est transmis à la jante et au pneu.

D'habitude en effet, c'est la nappe de rayons située à proximité des pignons qui transmet la majeure partie de ce couple. Ici, au contraire, une partie, et, de préférence la majeure partie, du couple est transmis par la nappe de rayons opposée aux pignons. Cette partie traverse le corps de moyeu et génère une torsion qui varie avec le couple transmis.

L'invention sera mieux comprise en se référant à la description ci-dessous et aux dessins en annexe qui en font partie intégrante.

La figure 1 représente une roue arrière de cycle vue de face.

La figure 2 est la roue de la figure 1 vue du côté des pignons.

La figure 3 est la roue de la figure 1 vue du côté opposé aux pignons.

La figure 4 représente en vue de face le moyeu de la roue selon un mode particulier de mise en oeuvre de l'invention.

La figure 5 représente en coupe le corps du moyeu de la figure précédente.

La figure 6 est une vue en coupe du moyeu selon le plan DD repéré en figure 5.

La figure 7 est une vue en coupe du corps de moyeu selon la vue HH repérée en figure 5.

La figure 8 est une vue du corps de moyeu selon la coupe FF repéré en figure 5.

La figure 9 est une vue semblable à la figure 8 qui illustre le fonctionnement du dispositif multiplicateur en charge.

La figure 10 représente en une vue de côté, de façon agrandie, la came du capteur associée aux moyens multiplicateur.

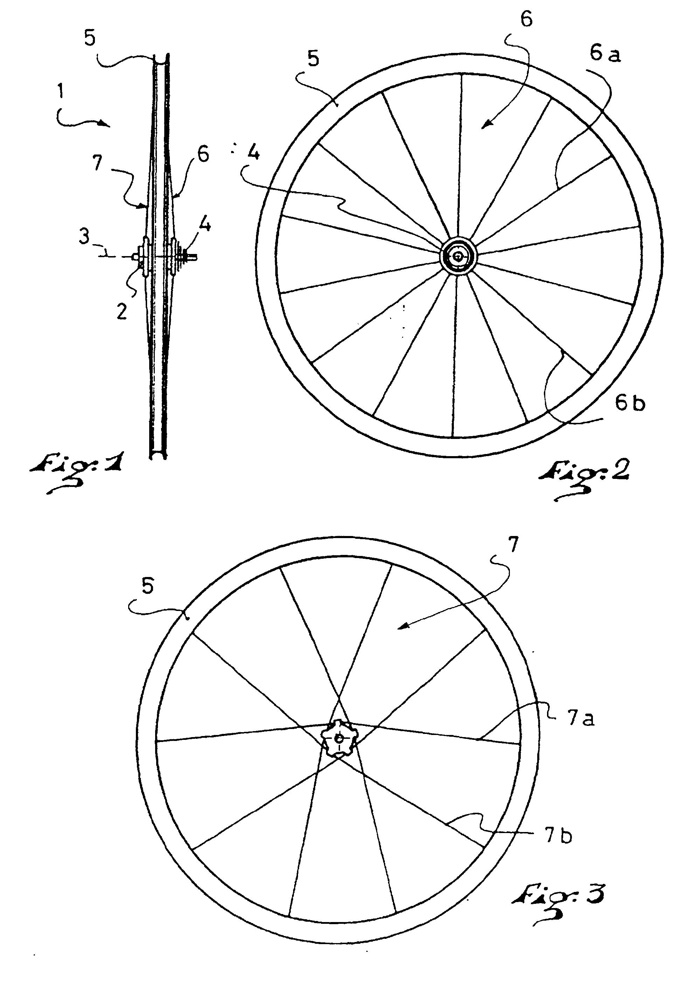

La figure 1 représente à titre d'illustration de l'invention une roue 1. Cette roue comprend un moyeu central 2 définissant un axe de révolution transversal 3.

D'un côté du moyeu se trouve un boîtier 4 de pignons qui sont prévus pour servir de renvoi à une chaîne d'entraînement. La roue comprend par ailleurs une jante annulaire 5 centrée sur l'axe 3. La jante est reliée aux deux extrémités du moyeu par deux nappes de rayons, respectivement 6 et 7.

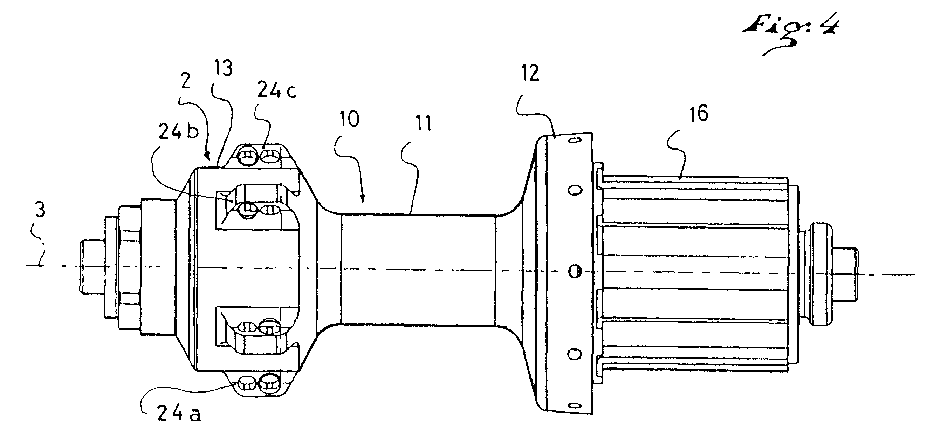

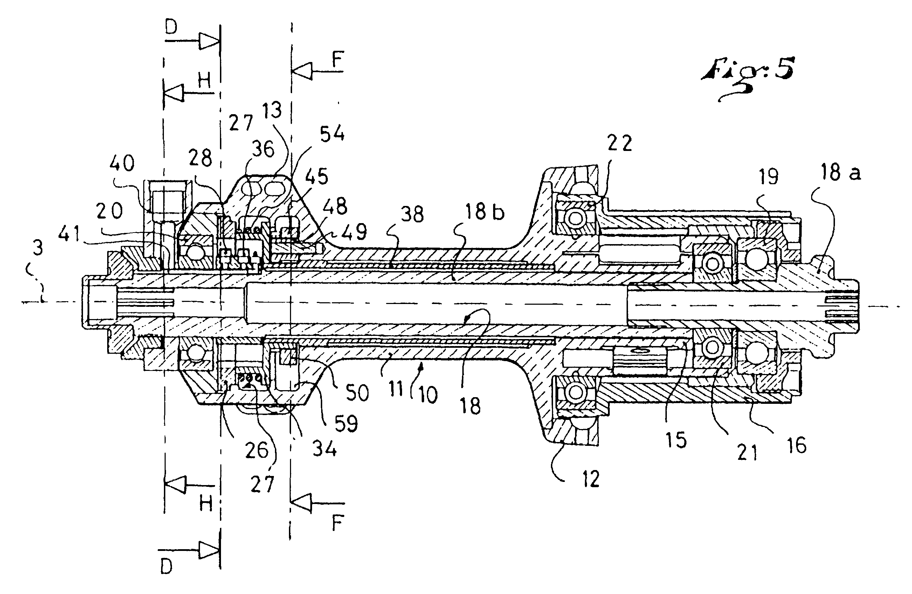

La figure 2 représente au premier plan les rayons 6a, 6b, ... de la nappe de rayons 6 située du côté du boîtier 4 de pignons. La figure 3 représente l'autre côté de la roue, avec en premier plan les rayons 7a, 7b, de la nappe de rayons 7.

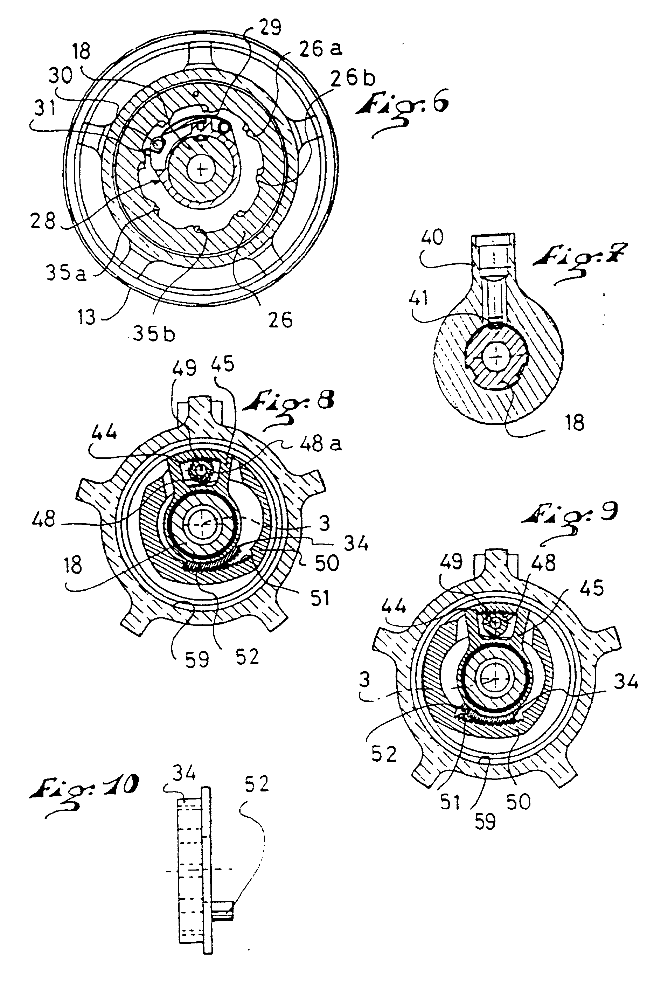

En se référant aux figures 4 et 5, le moyeu 2 comprend un corps de moyeu 10. Le corps de moyeu 10 présente approximativement la forme d'un diabolo centré sur l'axe 3. Il présente une partie médiane 11 de diamètre relativement petit, et deux joues latérales ou renflements 12 et 13 de diamètre supérieur. Ces joues sont prévues pour fournir des points d'ancrage aux différents rayons des nappes.

A l'une de ses extrémités, le corps de moyeu présente un embout 15 destiné à recevoir un corps de roue libre 16. Extérieurement, le corps de roue libre présente une surface cylindrique cannelée qui est prévue pour recevoir le boîtier de pignons. Un mécanisme de roue libre unidirectionnelle assure la liaison entre le corps de moyeu et le corps de roue libre. Un tel mécanisme est connu, par exemple d'après la demande de brevet publiée sous le numéro EP 0 703 376, et ne sera pas décrit en détail. A ce niveau, tout mécanisme approprié de roue libre peut convenir. On pourrait également construire le corps de moyeu d'une autre façon, afin qu'il soit prévu pour recevoir un mécanisme de roue libre indépendant, qui est rapporté à l'une de ses extrémités. Ces différents montages sont connus de l'homme du métier.

Le corps de moyeu et le corps de roue libre sont montés libres en rotation autour d'un arbre transversal 18 centré sur l'axe 3. L'arbre 18 est prévu pour être monté de façon solidaire sur un cadre de cycle. Par exemple, l'arbre 18 est creux, et l'assemblage est réalisé par une tige de blocage rapide d'un type connu. Des roulements 19, 20 sont situés à chacune des extrémités de l'arbre, et entre le corps de moyeu et l'arbre. D'autres roulements 21, 22 se trouvent entre le corps de roue libre, l'arbre transversal et le corps de moyeu.

L'arbre transversal 18 est ici en deux parties 18a et 18b, assemblées l'une à l'autre par des parties correspondantes taraudées et filetées. Des rondelles épaulées sont prévues aux extrémités des parties 18a, et 18b pour prendre appui contre les roulements externes. Les deux parties 18a, 18b sont vissées et bloquées. Un écrou comprenant l'une des rondelles épaulées est monté sur l'une des parties à son extrémité, et permet de régler le jeu de fonctionnement par son serrage . Des rondelles d'étanchéité ou tout autre moyen approprié est par ailleurs prévu à chacune des ouvertures du corps de moyeu.

Selon l'invention, le corps de moyeu est conformé en corps d'épreuve déformable en torsion autour de l'axe 3 sous l'action du couple d'entraînement que la chaîne transmet au pignon en prise et au corps de roue libre. En outre, des capteurs sont prévus pour mesurer le décalage angulaire entre les deux extrémités du corps de moyeu.

En se référant aux figures 4 et 5, la partie médiane 10 du corps de moyeu présente un diamètre externe relativement petit, et une épaisseur de paroi relativement fine. En outre, le corps de moyeu est réalisé en un matériau tel qu'un alliage d'aluminium. On a obtenu de bons résultats avec un alliage d'aluminium connu sous la référence 2014 T6 ou 7075 T6. Ces matériaux présentent une bonne élasticité, une limite élastique élevée. De plus; un alliage d'aluminium a l'avantage d'être léger. Naturellement tout autre matériau approprié convient.

On a obtenu de bons résultats avec une partie médiane présentant une longueur de 35 à 40 millimètres environ, un diamètre externe de 20 millimètres environ, et une épaisseur de paroi de 2 millimètres environ. Naturellement ces chiffres n'ont qu'une valeur indicative. De préférence, à la jonction entre les extrémités de la partie médiane et les joues, le corps de moyeu présente un rayon relativement important, ou une variation progressive de diamètre. Ceci évite les zones de rupture.

Pour solliciter le corps de moyeu à la torsion, les deux nappes de rayons sont prévues pour transmettre le couple d'entraînement du moyeu à la jante selon une répartition déterminée, qui, de préférence, ne varie pas avec l'intensité du couple ou la tension des rayons.

Selon le mode de répartition adopté pour le mode de réalisation illustré, la majeure partie du couple est transmis à la jante par la nappe de rayons 7 située du côté opposé aux pignons. Ainsi, cette partie du couple traverse le corps de moyeu, et génère une torsion qui est mesurée par des capteurs.

Pour ce faire, comme cela est visible dans la figure 2, la nappe de rayons 6 située du côté des pignons est formée par des rayons 6a, 6b orientés de façon radiale, ou quasi radiale compte tenu de l'inclinaison des rayons en direction du plan défini par la jante. La joue 12 prévue pour l'accrochage de ces rayons présente la forme d'une coupelle ouverte du côté de l'embout 15. La coupelle peut être une pièce indépendante rapportée sur le corps de moyeu et indexée, c'est-à-dire immobilisée dans une position angulaire précise, par exemple par une goupille, ou par un encastrement. La paroi de la coupelle est percée d'un pluralité d'orifices dans lesquels les rayons sont enfilés pour être retenus par leur tête. Les rayons utilisés ici sont des rayons de type droit, c'est-à-dire avec la tête dans le prolongement de la tige. Ces rayons sont réputés pour supporter une tension élevée. On a obtenu de bons résultats avec une nappe 6 formé de dix rayons radiaux.

Un tel mode de rayonnage est adapté pour assurer la liaison entre la jante et le moyeu. Par contre, compte tenu de l'orientation radiale des rayons, il n'est pas adapté pour transmettre un couple d'entraînement.

A l'inverse, la nappe 7 présente un rayonnage croisé. Les rayons 7a, 7b, de la nappe 7 sont inclinés par rapport à une direction radiale, pour favoriser la transmission du couple vers la jante. Ceci est visible dans la figure 3.

En se référant aux figures 4 et 5, la joue 13 présente une pluralité d'oreilles 24a, 24b, 24c...orientées de façon radiale. Chacune des oreilles présente deux orifices dans lesquels deux rayons sont enfilés tête-bêche pour être retenus par leur tête respective. Les rayons sont également des rayons droits. On a obtenu de bons résultats avec une nappe 7 formée de dix rayons 7a, 7b, ....reliés à cinq oreilles 24a, 24b ....

Naturellement, ces chiffres ne sont donnés qu'à titre indicatif, et le nombre de rayons de chacune des nappes pourrait être plus ou moins élevé selon l'utilisation prévue de la roue, notamment selon qu'il s'agit d'une roue pour un vélo de route ou un vélo tout terrain.

Pour la roue qui vient d'être décrite, le couple d'entraînement se répartit globalement de la façon suivante, 95% environ du couple est transmis par la nappe 7, et le reste, soit 5% environ par la nappe 6. La partie du couple transmise par la nappe 6 n'est pas exactement linéaire avec l'intensité du couple, à cause de l'augmentation de tension des rayons qui se produit pour des couples élevés. La répartition dépend également de la tension initiale des rayons. Toutefois, cette non-linéarité est peu marquée compte tenu du mode de rayonnage adopté. A ce sujet, on a remarqué qu'un rayonnage croisé des deux nappes présentait une sensibilité plus élevée à la tension initiale des rayons

En outre la proportion du couple transmis par la nappe 6 est très faible, si bien que l'on peut considérer globalement que cette répartition est constante sur une plage de couple d'entraînement correspondant à l'utilisation normale d'une bicyclette. Ainsi, on peut considérer que le corps de moyeu est soumis à un couple de torsion proportionnel au couple d'entraînement. A titre indicatif, on a estimé que dans cette plage, le couple pouvait varier entre 0 et 250 N.m (newtons-mètres) environ. A titre indicatif, pour une telle plage de valeurs, la torsion du corps de moyeu peut varier entre zéro et deux degrés et demi à trois degrés d'angle mesuré entre les deux extrémités du corps de moyeu, c'est-à-dire au niveau des deux joues 12 et 13.

Selon une variante de réalisation, la coupelle présente une couronne indépendante à laquelle les rayons de la nappe 6 sont accrochés et qui peut pivoter librement par rapport au reste du corps de moyeu. De préférence, dans ce cas, cette couronne indépendante est reliée au corps de moyeu par un roulement ou autre moyen similaire assurant une libre rotation. Ce mode de construction est adapté par exemple pour une roue à disques pleins, où on ne peut pas maîtriser la répartition du couple entre les deux nappes par le mode de rayonnage, pour la simple raison que ce sont les disques et non des rayons qui relient le moyeu et la jante.

De cette façon, on augmente encore la proportion du couple qui est transmise à la nappe 7 par le corps de moyeu.

Selon ce qui vient d'être décrit, on comprend que l'on recherche à transmettre par le corps de moyeu la plus grande partie du couple. Ceci n'est pas limitatif, et toute répartition pourrait convenir, avec une partie du couple plus ou moins importante transmise par la nappe 6, pourvu que la nappe de rayons 7 transmette au moins une partie non nulle de ce couple, cette partie traversant le corps de moyeu 12.

Autant que possible, la répartition est constante sur la plage de couple considéré, c'est-à-dire que le corps de moyeu est soumis à un couple de torsion proportionnel au couple d'entraînement. Selon une autre possibilité, on adopte une répartition donnée à priori non-linéaire, on étalonne pour cette répartition l'angle de torsion par rapport au couple d'entraînement, et on corrige la non-linéarité par un traitement numérique, au niveau d'une unité de traitement qui sera décrite plus loin.

L'angle de torsion entre les deux extrémités du corps de moyeu, globalement entre les deux joues 12 et 13, est mesuré au moyen de deux capteurs reliés à chacune des extrémités. Chacun des capteurs comprend une came en forme de bague, qui est liée en rotation avec l'extrémité du corps de moyeu à laquelle elle est associée, et un rupteur fixe monté sur l'arbre transversal. Ainsi, la figure 6 représente une came annulaire 26 montée par encastrement dans un logement 27 situé à l'intérieur du corps de moyeu, au niveau de sa joue 13. En direction de l'arbre transversal 18, la came présente une pluralité de dents 26a, 26b... qui sont réparties de façon régulière à la surface interne de la came. Un rupteur 28 est monté sur l'arbre 18 en regard de la came 26. Ce rupteur comprend une lamelle métallique 29 dont une extrémité libre est en appui contre un plot conducteur 30. Au-delà du plot, la lamelle présente une extrémité en forme d'anse 31 qui s'étend en direction des dents de la came. A chaque passage d'une dent, l'anse est soulevée, ce qui coupe la liaison électrique entre la lamelle et le plot. Après le passage de la dent, le contact électrique est rétabli entre la lamelle et le plot.

Un capteur semblable est associé à l'autre extrémité du corps de moyeu. Ce capteur présente une came 34 de même nature que la came 26, présentant des dents identiques réparties de façon identique. La figure 6 représente les dents 35a, 35b ... de la came 34. Un rupteur 36 semblable au rupteur 28 est par ailleurs monté sur l'arbre 18 en regard de la came 34. De préférence, pour un couple nul, les deux cames sont calées par rapport à leur rupteur respectif de façon qu'il existe un léger décalage initial entre l'ouverture des deux rupteurs. La mesure de torsion du corps de moyeu consiste à mesurer la variation de ce décalage au passage de chacune des dents sur le rupteur. Le nombre de dents de chaque came détermine le nombre de fois où la mesure est réalisée au cours d'un tour complet. La figure 6 montre huit dents, ce nombre n'est cependant pas limitatif et tout nombre inférieur ou supérieur convient.
Ainsi que cela est visible dans la figure 5, de préférence, les deux capteurs sont côte à côte. Ils sont situés du côté opposé aux pignons dans le logement 27 situé à l'extrémité du corps de moyeu, au niveau de la joue 13. Un tube 38 ramène par ailleurs de ce côté l'information sur la position angulaire de l'autre extrémité du corps de moyeu. Le tube 38 traverse le corps de moyeu sur une grande partie de sa longueur. Il s'étend entre l'arbre 18 et la partie médiane 11 du corps de moyeu. Du côté de la joue 12, il présente une extrémité encastrée dans le corps de moyeu. De préférence, l'encastrement est réalisé par un collage, ou toute autre technique appropriée qui permet de caler angulairement le tube 38 autour de l'axe 3. Ceci permet de régler le déphasage initial à une valeur déterminée. Le tube 38 ne transmet quasiment aucun effort. Il présente de ce fait une paroi très fine, et il est réalisé en un matériau léger, par exemple un alliage d'aluminium, de préférence dans un alliage connu sous le nom commercial de "ZAMAC". Cet alliage de zinc, d'aluminium et de manganèse, présente l'avantage d'avoir un module élastique supérieur à celui d'un alliage d'aluminium. Les cames sont quant à elles réalisées en une matière plastique.

Ainsi, contrairement aux dispositifs connus, c'est ici le corps de moyeu qui est le corps d'épreuve soumis à une torsion. Le tube 38 ne fait que ramener une information angulaire vers une extrémité du moyeu.

Des fils électriques de connexion non représentés dans les figures relient par ailleurs les lamelles et les plots des rupteurs à une prise de raccordement qui est logé dans le canon 40 situé dans la partie extrême de l'arbre central.

La figure 7 représente une section transversale du moyeu à ce niveau. Le canon est porté par un élément 41 rapporté, et assemblé au reste du corps de moyeu avec l'assemblage des deux parties 18a et 18b de l'arbre 18. Une rainure 41 pour le passage des fils est visible dans cette figure.

L'angle de torsion entre les deux extrémités du corps de moyeu étant relativement faible, l'invention propose de mesurer cet angle au travers de moyens multiplicateur qui vont maintenant être décrits. Dans le cas présent ces moyens sont interposés entre la joue 12, par l'intermédiaire du tube 38, et la came 34 qui lui est associée.

Les moyens multiplicateur comprennent une première portion de roue dentée 44 portée par un élément central 45 assemblé à l'extrémité libre du tube 38, ou réalisés de façon monobloc avec le tube. La portion 44 est orientée vers l'axe 3, elle est circulaire et centrée sur cet axe.

La portion 44 est en prise avec la denture 48a d'un pignon de petit diamètre 48 montée pivotante autour d'un axe 49. L'axe est porté par l'extrémité du corps de moyeu situé du côté de la joue 13, c'est-à-dire en quelque sorte, l'extrémité de référence de la mesure par rapport à laquelle le déphasage angulaire de l'autre extrémité est mesuré. L'axe 49 tourne autour de l'axe 3 avec la rotation de la'roue.

Le pignon 48 est solidaire d'une sorte de balancier 50 qui s'étend depuis la zone de l'axe 49, jusqu'au delà de l'axe transversal 3. Le balancier 50 pivote autour de l'axe 3 avec la roue autour de l'arbre transversal, et autour de l'axe 49 avec la rotation du pignon 48, c'est-à-dire avec la torsion du corps de moyeu.

Le balancier 50 porte du côté opposé au pignon 48 une portion de roue dentée 51 orientée en direction de l'axe 3. Cette portion est en prise avec une autre portion 52 qui est située à la périphérie externe de la came 34. Comme cela est visible dans la figure 10, la portion 52 de la came 34 est déportée en direction de l'autre extrémité du moyeu. Ainsi, la rotation du balancier 50 autour de l'axe 49 entraîne à son tour la came 34 en rotation autour de l'arbre transversal 18. Un ressort cylindrique de torsion 54 exerce sur la came 34 un moment de rappel élastique en direction de la position initiale de la came. Ce ressort assure le rappel élastique de la came, ainsi que le rattrapage des jeux.

L'angle de torsion du corps de moyeu est ainsi multiplié dans un rapport lié au rapport des rayons des différentes portions de roue dentée, et de la position relative des axes de rotation. A titre indicatif, on a réussi de cette façon à atteindre un rapport de multiplication d'une valeur de treize.

En se référant aux figures, la figure 8 représente la position relative des moyens multiplicateur pour un couple nul, et la figure 9 représente ces mêmes moyens pour un couple de l'ordre de 240 N.m. Comme cela est visible dans la figure 5, la came 34 et la came 26 sont en appui chacune des nervures annulaires qui délimitent des chambres successives pour les cames et les moyens multiplicateur. La chambre 59 en particulier est prévue pour permettre le débattement angulaire du balancier 50 autour de l'axe 49 sur toute la plage de mesure du couple de torsion,

Les différents éléments des moyens multiplicateur sont réalisés en tout matériau approprié. Avantageusement, ils sont réalisés en un alliage connu sous le nom commercial de "ZAMAC" qui est réputé pour réaliser par moulage des pièces d'une grande précision, et pour présenter un modulé élastique élevé.

Le moyeu qui vient d'être décrit est prévu pour être connecté à une unité de traitement. L'ouverture et la fermeture successive des rupteurs génèrent des impulsions électriques. Ces impulsions renseignent sur la vitesse pure de rotation de la roue par leur vitesse de succession, et sur le couple d'entraînement exercé par la chaîne, par leur déphasage.

L'unité de traitement est en mesure de calculer la puissance développée par le cycliste en traitant la mesure de torsion par rapport au temps. Cette unité de traitement comprend notamment des moyens de lissage des signaux recueillis par les capteurs. Le couple est en effet susceptible de varier de façon significative au cours d'un tour de pédalier. Au niveau de l'unité de traitement, la puissance instantanée est mesurée d'après la formule P = Cω, où P désigne la puissance en watts, C le couple en N.m, et où la vitesse angulaire en rd/s (radian par seconde). La vitesse angulaire est directement déductible de la mesure du couple. Il suffit de mesurer l'écart de temps entre le passage de deux cames successives de l'un des capteurs. L'énergie développée par le cycliste est mesurée en intégrant la mesure de puissance par rapport au temps, c'est-à-dire en appliquant la formule E = ∫Pdt, où E est l'énergie en watts-secondes.

L'unité comprend par ailleurs tout moyen de traitement approprié, par exemple d'affichage instantané, d'affichage d'une valeur moyenne, de comparaison avec une valeur de consigne, de mémorisation de valeurs de consigne, d'enregistrement de la mesure de couple, de restitution des valeurs enregistrées. Eventuellement, tel que cela a été évoqué plus haut, l'unité de traitement peut présenter un filtre, notamment un filtre numérique pour corriger une non-linéarité éventuelle de la mesure de couple.

L'unité de traitement peut être combinée aussi à une unité générale de gestion électronique des performances du cycle et du cycliste.

Naturellement, la construction qui vient d'être décrite est susceptible d'être modifiée. En particulier, la position des capteurs du côté opposé à l'embout est avantageuse pour avoir une connexion électrique située du coté opposé au dérailleur arrière. Cette positon n'est pas limitative, et les capteurs pourraient être situés du côté de l'embout 15.

Les capteurs pourraient également être disposés vers chacune des extrémités du moyeu.

La liaison entre le moyeu et la jante par des rayons n'est pas limitative, et tout autre moyen approprié convient, par exemple des bâtons, des disques.

D'autres moyens pourraient aussi convenir pour multiplier la valeur du décalage angulaire entre les deux extrémités du moyeu.

Enfin, l'invention n'est pas limitée à une roue à deux nappes de rayons. Elle concerne également les roues où la liaison entre le moyeu et la jante comprend des disques, des bâtons ou une partie centrale en bulbe ou lenticulaire. Comme cela a été évoqué plus haut, dans ce cas, il n'est pas possible à priori de maîtriser la répartition de couple entre les deux côtés du moyeu.

Pour remédier à cela, du côté des pignons, une solution consiste à isoler par un roulement, ou autre moyen similaire, le moyeu et le reste de la roue, si bien que l'intégralité ou la quasi-intégralité du couple traverse le moyeu pour atteindre la jante.

## Revendications

**1.** Moyeu de roue, notamment de roue de cycle, comprenant un arbre transversal (18) prévu pour être assemblé de façon solidaire sur un cadre, et définissant un axe transversal de révolution (3), un corps de moyeu (10) monté en rotation libre autour de l'arbre transversal (18), le corps de moyeu ayant une partie médiane (11), deux joues latérales (12, 13), les joues latérales ayant des moyens d'accrochage prévus pour des moyens de liaison avec une jante, le corps de moyeu ayant au-delà de l'une des joues un embout latéral (15) sur lequel est monté un mécanisme de roue libre prévu pour recevoir au moins un pignon d'entraînement,
**caractérisé par le fait que** la partie médiane (11) du corps de moyeu est conformée en corps d'épreuve déformable en torsion autour de l'axe transversal de révolution (3), et un couple de deux capteurs (26, 34, 28, 36) est disposé dans le moyeu, chacun associé à une extrémité du moyeu vers chacune des joues (12, 13), les capteurs étant prévus pour mesurer au moins de façon séquentielle le décalage angulaire des deux extrémités du corps de moyeu (11) par rapport à l'axe transversal de révolution (3).

**2.** Moyeu selon la revendication 1, **caractérisé par le fait que** la joue (12) située du côté de l'embout (15) est prévue pour recevoir des rayons (6a, 6b) orientés de façon radiale.

**3.** Moyeu selon la revendication 1, **caractérisé par le fait que** le joue (12) située du côté de l'embout (15) présente une couronne d'accrochage prévue pour recevoir les rayons, et que la couronne est montée libre en rotation par rapport au corps de moyeu.

**4.** Moyeu selon la revendication 1, **caractérisé par le fait que** les deux capteurs sont côte à côte, qu'ils sont situés dans la zone d'une joue (13), dite première joue, que l'un d'eux comprend une came (34) montée à l'extrémité libre d'un tube (38) coaxial avec l'axe transversal (3), intercalé entre l'arbre (18) et le corps de moyeu (10), l'extrémité du tube opposée à la came étant reliée solidairement au corps de moyeu (10) dans la zone de l'autre joue (12), dite seconde joue.

**5.** Moyeu selon la revendication 4, **caractérisé par le fait que** la première joue (13) est la joue située du côté opposé à l'embout (15).

**6.** Moyeu selon la revendication 4, **caractérisé par le fait que** le tube (38) est relié solidairement au corps de moyeu (10) par encastrement et collage.

**7.** Moyeu selon la revendication 4, **caractérisé par le fait que** chacun des capteurs comprend une came (26, 34) centrée sur l'arbre transversal (18), que l'une des cames dite première came (26) est montée fixe par rapport à l'extrémité du corps de moyeu (10) située du côté de la première joue (13), que l'autre came (34), dite seconde came est montée pivotante par rapport à l'extrémité du moyeu situé du côté de la seconde joue (12), et que la position angulaire de la seconde came (34) est pilotée par un train multiplicateur de portions de roues dentées (44, 48, 51, 52) dont les axes de rotation sont supportés par l'extrémité du corps de moyeu situé du côté de la première joue (13) ou bien sont concentriques avec l'axe transversal (3), et qui reçoit en entrée la position angulaire de l'extrémité du corps de moyeu (10) situé du côté de la seconde joue (12).

**8.** Moyeu selon la revendication 7, **caractérisé par le fait que** le train multiplicateur de portions de roues dentées comprend une première portion (44) de roue dentée de grand diamètre, dont la denture est orientée vers l'arbre central (18), et qui est reliée solidairement à l'extrémité libre du tube (38), un balancier (50), articulé en rotation autour d'un axe de support (49) porté par l'extrémité du corps de moyeu (10) situé du côté de la première joue (13), l'axe de support (49) étant excentré par rapport à l'axe transversal (3), et situé à proximité de la première portion (44) de roue dentée, que le balancier (50) porte de façon solidaire un pignon (48) de petit diamètre, centré sur l'axe de support, et dont la denture (48a) est prévue pour s'engrener sur les dents de la première portion (44) de roue dentée, que dans la zone du balancier (50) opposée à la roue dentée de petit diamètre, se trouve une seconde portion (51) de roue dentée orientée vers l'axe transversal (3), qui est prévue pour s'engrener avec une troisième portion (52) de roue dentée située à la périphérie externe d'une portion déportée de la seconde came (34).

**9.** Moyeu selon la revendication 4, **caractérisé par le fait que** chacune des cames présente une portion en forme de bague qui présente à sa périphérie interne au moins une dent (26a, 26b, 35a, 35b) prévue pour actionner un rupteur (28, 36) monté sur l'arbre transversal (18).

**10.** Roue de cycle comprenant un moyeu central (2) avec un arbre transversal (18) définissant un axe transversal (3), un corps de moyeu (10) monté en rotation libre autour de l'arbre (18), avec une partie médiane (11) et deux joues latérales (12, 13) et un embout (15) prévu pour recevoir au moins un pignon de transmission et un mécanisme de roue libre unidirectionnelle, une jante (4), et deux nappes (6, 7) de rayons reliant la jante (4) à chacune des joues (12, 13) du moyeu, **caractérisée par le fait que** la partie médiane (10) du moyeu est conformée comme un corps d'épreuve déformable en torsion, que les nappes (6, 7) de rayons sont prévues pour que la nappe (7) de rayons située du côté opposé à l'embout (15) soit destinée à transmettre une partie déterminée du couple d'entraînement transmis à l'embout par le pignon et le mécanisme de roue libre, et que le moyeu (2) présente des capteurs (26, 34, 28, 36) prévus pour mesurer la position angulaire relative des extrémités du corps de moyeu situées vers chacune de ses joues.

**12.** Roue selon la revendication 11, **caractérisée par le fait que** la nappe (6) de rayons située du côté de l'embout (15) présente des rayons (6a, 6b) orientés de façon radiale.

**13.** Roue selon la revendication 11, **caractérisée par le fait que** la nappe (6) de rayons située du côté de l'embout (15) est reliée à une bague montée en rotation libre par rapport au corps de moyeu, autour de l'axe transversal.

**14.** Roue de cycle comprenant
- un moyeu central (2) avec un arbre transversal (18) définissant un axe transversal (3), un corps de moyeu (10) monté en rotation libre autour de l'arbre (18), avec une partie médiane (11) et deux joues latérales (12, 13) et un embout (15) prévu pour recevoir au moins un pignon de transmission et un mécanisme de roue libre unidirectionnelle,
- une jante (4),
- et une liaison reliant la jante (4) à chacune des joues (12, 13) du moyeu,
**caractérisé par le fait qu'**il présente un moyeu (2) selon l'une quelconque des revendications 1 à 10 précédentes.

**15.** Roue de cylce selon la revendication 14, **caractérisée par le fait que** du côté de l'embout (15), un roulement est prévu entre le moyeu et le reste de la roue pour isoler ces deux éléments l'un de l'autre.

## Patentansprüche

**1.** Radnabe, insbesondere eines Zweiradrads, aufweisend eine transversale Welle (18), die vorgesehen ist, in fest verbundener Weise an einem Rahmen montiert zu sein und eine transversale Drehachse (3) definierend, einen Nabenkörper (10), welcher frei in Drehung um die transversale Welle (18) montiert ist, wobei der Nabenkörper einen mittleren Teil (11) und zwei seitliche Backen (12, 13) aufweist, wobei die seitlichen Backen Einhakmittel aufweisen, die für die Verbindungsmittel mit einer Felge vorgesehen sind, wobei der Nabenkörper oberhalb einer der Backen einen seitlichen Ansatz (15) aufweist, auf welchem ein Freilaufmechanismus montiert ist zum Aufnehmen von mindestens einem Antriebszahnrad,
**dadurch gekennzeichnet, dass** der mittlere Teil (11) des Nabenkörpers angepasst ist als in Torsion um die transversale Drehachse (3) deformierbarer Probekörper und ein Paar von zwei Messfühlern (26, 34, 28, 36) in der Nabe angeordnet ist, wobei jeder an einem Ende der Nabe in Richtung zu jeder der Backen (12, 13) angefügt ist, wobei die Messfühler vorgesehen sind mindestens in sequentieller Weise die winkelmäßige Verschiebung der zwei Enden des Nabenkörpers (11) in Bezug zu der transversalen Drehachse (3) zu messen.

**2.** Nabe nach Anspruch 1, **dadurch gekennzeichnet, dass** die Backe (12), welche auf der Seite des Ansatzes (15) angeordnet ist, zum Aufnehmen von Speichen (6a, 6b) vorgesehen ist, welche in radialer Weise ausgerichtet sind.

**3.** Nabe nach Anspruch 1, **dadurch gekennzeichnet, dass** die Backe (12), welche auf der Seite des Ansatzes (15) angeordnet ist, eine Einhakkrone aufweist, die vorgesehen ist zum Aufnehmen von Speichen und dass die Krone frei in Drehung in Bezug auf den Nabenkörper montiert ist.

**4.** Nabc nach Anspruch 1, **dadurch gekennzeichnet, dass** die zwei Messfühler Seite an Seite sind, dass sie in einer Zone einer Backe (13) angeordnet sind, genannt erste Backe, dass der eine von ihnen eine Nocke (34) aufweist, die an dem freien Ende eines mit der transversalen Achse (3) koaxialen Rohres (38) montiert ist, zwischengeschaltet zwischen der Welle (18) und dem Nabenkörper (10), wobei das Ende des Rohrs, welches zu der Nocke gegenüberliegend ist, fest mit dem Nabenkörper (10) verbunden ist in der Zone der anderen Backe (12), genannt zweite Backe.

**5.** Nabe nach Anspruch 4, **dadurch gekennzeichnet, dass** die erste Backe (13) die Backe ist, welche auf der Seite gegenüberliegend zum dem Ansatz (15) angeordnet ist

**6.** Nabe nach Anspruch 4, **dadurch gekennzeichnet, dass** das Rohr (38) fest mit dem Nabenkörper (10) durch festes Einspannen und Kleben verbunden ist.

**7.** Nabe nach Anspruch 4, **dadurch gekennzeichnet, dass** jeder der Messfühler eine Nocke (26, 34) aufweist, welche auf der transversalen Achse (18) zentriert ist, dass eine der Nocken, genannt erste Nocke (26), fest in Bezug auf das Ende des Nabenkörpers (10) montiert ist, welches auf der Seite der ersten Backe (13) angeordnet ist, dass die andere Nocke (34), genannt zweite Nocke, schwenkend in Bezug auf das Ende der Nabe montiert ist, welches auf der Seite der zweiten Backe (12) angeordnet ist, und dass die Winkelposition der zweiten Nocke (34) durch ein Multiplikatorgestell von Abschnitten von mit Zähnen versehenen Rädern (44, 48, 51, 52) gesteuert wird, deren Drehachsen durch das Ende des Nabenkörpers getragen werden, welches auf dem Ende der ersten Backe (13) angeordnet ist oder aber konzentrisch mit der transversalen Achse (3) sind und welche eingangs die Winkelposition des Endes des Nabenkörpers (10) empfängt, welches auf der Seite der zweiten Backe (12) angeordnet ist

**8.** Nabe nach Anspruch 7, **dadurch gekennzeichnet, dass** das Multiplikatorgestell der Abschnitte des gezahnten Rads einen ersten Abschnitt (44) des gezahnten Rads mit großem Durchmesser aufweist, dessen Zahnung in Richtung zu der zentralen Welle (18) gerichtet ist, und welcher fest mit dem freien Ende des Rohrs (38) verbunden ist, einem Schwinghebel (50), welcher in Drehung um eine Stützwelle (49) angelenkt ist, welche durch das Ende des Nabenkörpers (10) getragen wird, welches auf der Seite der ersten Backe (13) angeordnet ist, wobei die Stützwelle (49) dezentriert ist in Bezug auf die transversale Achse (3) und in der Nähe des ersten Abschnitts (44) des gezahnten Rads angeordnet ist, dass der Schwinghebel (50) in fest verbundener Weise ein kleines Zahnrad (58) mit kleinem Durchmesser aufweist, das auf der Stützwelle zentriert ist und dessen Zahnung (48a) vorgesehen ist, in den Zähnen des ersten Abschnitts (44) des gezahnten Rads einzugreifen, dass in der Zone des Schwinghebels (50), die gegenüberliegend zu dem gezahnten Rad mit kleinem Durchmesser ist, sich ein zweiter Abschnitt (51) des gezahnten Rads befindet, welcher in Richtung zur transversalen Achse (3) orientiert ist, welcher vorgesehen ist, mit einem dritten Abschnitt (52) des gezahnten Rads, der an dem äußeren Umfang eines Abschnitts, der verschoben von der zweiten Nocke (34) ist, einzugreifen.

**9.** Nabe nach Anspruch 4, **dadurch gekennzeichnet, dass** jede der Nocken einen Abschnitt in Form eines Rings aufweist, welcher an seiner inneren Peripherie mindestens einen Zahn (26a, 26b, 35a, 35b) aufweist, der vorgesehen ist, einen Unterbrecher (28, 36), welcher auf der transversalen Welle (18) montiert ist, zu betätigen.

**10.** Rad eines Zweiradrads, aufweisend eine zentrale Nabe (2) mit einer transversalen Welle (18), wobei sie eine transversale Achse (3) definiert, einen Nabenkörper (10), welcher frei in Drehung um die Welle (18) montiert ist mit einem mittleren Teil (11) und zwei seitlichen Backen (12, 13) und einem Ansatz (15), der vorgesehen ist zum Aufnehmen von mindestens einem Übertragungszahnrad und einem uni-direktionellen Freilauhnechanismus, einer Felge (4) und zwei Schichten (6, 7) von Speichen, welche die Felge (4) mit jeder der Backen (12, 13) der Nabe verbinden, **dadurch gekennzeichnet, dass** der mittlere Teil (10) der Nabe angepasst ist, als ein in Torsion deformierbarer Probekörper, dass die Schichten (6, 7) der Speichen vorgesehen sind, damit die Schicht (7) der Speichen auf der Seite angeordnet ist, welche gegenüberliegend zu dem Ansatz (15) ist, dafür bestimmt ist, einen bestimmten Teil des Antriebsmoments, welcher an dem Ansatz durch das Zahnrad und den Freilaufmechanismus übertragen wird und dass die Nabe (2) zwei Messfühler (26, 34, 28, 36) aufweist, welche zum Messen der relativen Winkelposition der Enden des Nabenkörpers vorgesehen sind, welche in Richtung jeder der Backen angeordnet sind.

**12.** Rad nach Anspruch 11, **dadurch gekennzeichnet, dass** die Schicht (6) der Speichen, welche auf der Seite des Ansatzes (15) angeordnet ist, Speichen (6a, 6b) aufweist, die in radialer Weise orientiert sind.

**13.** Rad nach Anspruch 11, **dadurch gekennzeichnet, dass** die Schicht (6) der Speichen, welche auf der Seite des Ansatzes (15) angeordnet sind, mit einem Ring verbunden ist, welcher frei in Drehung in Bezug auf den Nabenkörper um die transversale Achse montiert sind.

**14.** Rad eines Zweirads aufweisend:
- eine zentrale Nabe (2) mit einer transversalen Welle (18), welche eine transversale Achse (3) definiert, einen Nabenkörper (10), der frei in Drehung um die Welle (18) montiert ist, mit einem mittleren Teil (11) und zwei seitlichen Backen (12, 13) und einem Ansatz (15), der vorgesehen ist zumindest ein Übertragungszahnrad und einen uni-direktionellen Freilaufmechanismus zu empfangen,
- eine Felge (4)
- und eine Verbindung, welche die Felge (4) mit jeder der Backen (12, 13) der Nabe verbindet,
**dadurch gekennzeichnet, dass** es eine Nabe (2) gemäß irgendeinem der zuvorigen Ansprüche 1 bis 10 aufweist.

**15.** Rad eines Zweirads gemäß Anspruch 14, **dadurch gekennzeichnet, dass** auf der Seite des Ansatzes (15) ein Lager vorgesehen ist zwischen der Nabe und dem Rest des Rads zum Isolieren dieser zwei Elemente voneinander.

## Claims

**1.** Wheel hub, particularly for a bicycle wheel, having a transverse shaft (18) provided to be integrally assembled to a frame, and defining a transverse revolutionary axis (3), a hub body (10) mounted in free rotation about the transverse shaft (18), the hub body having a median portion (11), two lateral cheeks (12, 13), the lateral cheeks having hooking means provided for means for connecting with a rim, the hub body having, beyond one of the cheeks, a lateral end piece (15) on which is mounted a free wheel mechanism provided to receive at least one driving pinion,
**characterized in that** the median portion (11) of the hub body is shaped into a testing body that is deformable in torsion about the revolutionary transverse axis (3), and a couple of two sensors (26, 34, 28, 36) is arranged in the hub, each associated with one end of the hub towards each of the cheeks (12, 13), the sensors being provided to measure at least sequentially the angular displacement of the two ends of the hub body (11) with respect to the transverse revolutionary axis (3).

**2.** Hub according to claim 1, **characterized in that** the cheek (12) located on the side of the end piece (15) is provided to receive spokes (6a, 6b) oriented radially.

**3.** Hub according to claim 1, **characterized in that** the cheek (12) located on the side of the end piece (15) has a fastening ring provided to receive the spokes, and that the ring is mounted in free rotation with respect to the hub body.

**4.** Hub according to claim 1, **characterized in that** the two sensors are side by side, that they are located in the zone of a cheek (13), called a first cheek, that one of them has a cam (34) mounted at the free end of a tube (38) that is coaxial with the transverse axis (3), inserted between the shaft (18) and the hub body (10), the end of the tube opposite the cam being integrally connected to the hub body (10) in the zone of the other cheek (12), called a second cheek.

**5.** Hub according to claim 4, **characterized in that** the first cheek (13) is the cheek located on the side opposite the end piece (15).

**6.** Hub according to claim 4, **characterized in that** the tube (38) is integrally connected to the hub body (10) by nesting and gluing.

**7.** Hub according to claim 4, **characterized in that** each of the sensors has a cam (26, 34) centered on the transverse shaft (18), **in that** one of the cams, called a first cam (26), is permanently mounted with respect to the end of the hub body (10) located on the side of the first cheek (13), **in that** the other cam (34), called the second cam, is pivotally mounted with respect to the end of the hub located on the side of the second cheek (12), and that the angular position of the second cam (34) is controlled by a speed increase gear train having toothed wheel portions (44, 48, 51, 52) whose rotational axes are supported by the end of the hub body located on the side of the first cheek (13) or are concentric to the transverse axis (3), and which receives in entry the angular position of the end of the hub body (10) located on the side of the second cheek (12).

**8.** Hub according to claim 7, **characterized in that** the speed increase gear train having toothed wheel portions has a first portion (44) of a toothed wheel of large diameter, the teeth of which are oriented towards the central shaft (18), and which is integrally connected to the free end of the tube (38), a rocker (50), articulated in rotation about a support axis (49) borne by the end of the hub body (10) located on the side of the first cheek (13), the support axis (49) being off-centered with respect to the transverse axis (3), and located near the first portion (44) of the toothed wheel, **in that** the rocker (50) integrally bears a pinion (48) of small diameter, centered on the support axis, and the teeth (48a) of which are provided to mesh with the teeth of the first portion (44) of the toothed wheel, **in that** in the zone of the rocker (50) opposite the toothed wheel of small diameter is a second portion (51) of a toothed wheel oriented toward the transverse axis (3), which is provided to mesh with a third portion (52) of the toothed wheel located at the outer periphery of a portion that is displaced from the second cam (34).

**9.** Hub according to claim 4, **characterized in that** each of the cams has a portion in the shape of a ring that has, on its inner periphery, at least one tooth (26a, 26b, 35a, 35b) provided to actuate a breaker (28, 36) mounted on the transverse shaft (18).

**10.** Bicycle wheel having a central hub (2) with a transverse shaft (18) defining a transverse axis (3), a hub body (10) mounted in free rotation about the shaft (18), with a median portion (11) and two lateral cheeks (12, 13) and an end piece (15) provided to receive at least one transmission pinion and a unidirectional free wheel mechanism, a rim (4), and two sets (6, 7) of spokes connecting the rim (4) to each of the cheeks (12, 13) of the hub, **characterized in that** the median portion (10) of the hub is shaped like a testing body that is deformable in torsion, that the sets (6, 7) of spokes are provided so that the set (7) of spokes located on the side opposite the end piece (15) is adapted to be transmitted to a predetermined portion of the driving torque transmitted to the end piece by the pinion and the free wheel mechanism, and that the hub (2) has sensors (26, 34, 28, 36) provided to measure the relative angular position of the ends of the hub body located towards each of its cheeks.

**12.** Wheel according to claim 11, **characterized in that** the set (6) of spokes located on the side of the end piece (15) has spokes (6a, 6b) oriented radially.

**13.** Wheel according to claim 11, **characterized in that** the set (6) of spokes located on the side of the end piece (15) is connected to a ring mounted in free rotation with respect to the hub body, about the transverse axis,

**14.** Bicycle wheel having
- a central hub (2) with a transverse shaft (18) defining a transverse axis (3), a hub body (10) mounted in free rotation about the shaft (18), with a median portion (11) and two lateral cheeks (12, 13) and an end piece (15) provided to receive at least one transmission pinion and a unidirectional free wheel mechanism,
- a rim (4),
- and a connection joining the rim (4) to each of the cheeks (12, 13) of the hub,
**characterized in that** it has a hub (2) according to any of the preceding claims 1-10.

**15.** Bicycle wheel according to claim 14, **characterized in that** on the side of the end piece (15), a bearing is provided between the hub and the rest of the wheel to isolate these two elements from each other.
